# EUROPÄISCHE PATENTSCHRIFT

(11) **EP 1 012 191 B1**
(45) Veröffentlichungstag und Bekanntmachung des Hinweises auf die Patenterteilung: **11.04.2007**
(21) Anmeldenummer: 98903938.3
(22) Anmeldetag: 18.02.1998
(51) Int. Cl.: C07K 14/755, A61K 38/38

(54) **VERFAHREN ZUR GEWINNUNG VON HOCHREINEM vWF ODER FACTOR VIII/vWF-KOMPLEX**
METHOD FOR OBTAINING HIGH-PURITY VWF OR FACTOR VIII/VWF COMPLEX
PROCEDE PERMETTANT D'OBTENIR UN FACTEUR DE VON WILLEBRAND (vWF) OU UN COMPLEXE FACTEUR VIII

(30) Priorität: 27.02.1997 AT 33997
(43) Veröffentlichungstag der Anmeldung: 28.06.2000
(73) Patentinhaber: Baxter Aktiengesellschaft, 1221 Wien (AT)
(72) Erfinder: MITTERER, Artur, 2304 Orth/Donau (AT); FIEDLER, Christian, A-1100 Wien (AT); FISCHER, Bernhard, A-1160 Wien (AT); DORNER, Friedrich, A-1230 Wien (AT); EIBL, Johann, A-1180 Wien (AT)
(74) Vertreter: Weinzinger, Arnulf
(86) Internationale Anmeldenummer: PCT/AT1998/000033
(87) Internationale Veröffentlichungsnummer: WO 1998/038218

(56) Entgegenhaltungen:
- EP-A- 0 295 645
- EP-A- 0 705 846
- WO-A-97/34930
- WO-A-97/39033
- US-A- 4 774 323
- GROSS W. ET AL: 'Physiologische Chemie', 1989, VCH, WEINHEIM

## Beschreibung

Die Erfindung betrifft ein Verfahren zur Gewinnung von hochreinem vWF bzw. Faktor VIII/vWF-Komplex aus einem biologischen Ausgangsmaterial mittels Immunaffinitätschromatographie sowie eine stabile Präparation enthaltend hochreinen vWF oder Faktor VIII/vWF-Komplex.

Die Blutgerinnung ist ein komplexer Prozeß, der die sequentielle Interaktion einer Reihe von Komponenten, insbesondere von Fibrinogen, Faktor II, Faktor V, Faktor VII, Faktor VIII, Faktor IX, Faktor X, Faktor XI und Faktor XII einschließt. Der Verlust einer dieser Komponenten oder die Inhibierung seiner Funktionalität führt zu einer verstärkten Blutgerinnungsneigung, die bei einigen Patienten lebensbedrohlich sein kann.

Von Willebrand Faktor (vWF) zirkuliert im Plasma komplexiert mit Faktor VIII, wobei Faktor VIII die Blutgerinnung unterstützt und vWF im Komplex mit Faktor VIII diesen stabilisiert und vor proteolytischem Abbau schützt. Durch seine Funktion bei der Plättchenaggregation greift der vWF auch direkt in die Blutgerinnung ein. vWF existiert im Plasma in einer Serie von multimeren Formen von einem Molekulargewicht von 1 x 10⁶ bis 20 x 10⁶ Dalton. Der vWF ist ein Glykoprotein, welches vorwiegend in Endothelzellen von Säugern gebildet und anschließend in die Zirkulation freigesetzt wird. Dabei wird in den Zellen ausgehend von einer Polypeptidkette mit einem Molekulargewicht von ca. 220 kD durch Ausbildung von mehreren Schwefelbrücken ein vWF-Dimer mit einem Molekulargewicht von 550 kD gebildet. Aus den vWF-Dimeren werden dann durch Verknüpfung weitere Polymere des vWF mit immer höherem Molekulargewicht, bis zu 20 Millionen Dalton, hergestellt. Es wird vermutet, daß insbesondere den hochmolekularen vWF-Multimeren eine essentielle Bedeutung bei der Blutgerinnung zukommt.

Bei der Hämophilie ist die Blutgerinnung durch Mangel an bestimmten plasmatischen Blutgerinnungsfaktoren gestört. Bei der Hämophilie A beruht die Blutungsneigung auf einem Mangel an Faktor VIII bzw. einem Mangel an vWF, der einen wesentlichen Bestandteil des Faktor VIII darstellt. Die Behandlung der Hämophilie A erfolgt in erster Linie durch Ersatz des fehlenden Gerinnungsfaktors durch Faktorenkonzentrate, z.B. durch Infusion von Faktor VIII, Faktor VIII-Komplex oder vWF.

Das vWF-Syndrom besitzt mehrere Krankheitsbilder, die auf eine Unter- oder Überproduktion des vWFs zurückzuführen sind. So führt eine Überproduktion von vWF zur vermehrten Neigung zu Thrombosen. Eine Unterversorgung aufgrund der Abwesenheit oder einer Reduktion von hochmolekularen Formen des vWF manifestiert sich in einer vermehrten Blutungsneigung und einer verlängerten Blutungszeit durch die Inhibition der Plättchenaggregation und des Wundverschlußes.

Der Mangel an vWF kann auch eine phänotypische Hämophilie A hervorrufen, da der vWF ein wesentlicher Bestandteil des funktionellen Faktor VIII ist. In diesen Fällen ist die Halbwertszeit des Faktor VIII derart verringert, daß seine Funktion in der Blutgerinnungskaskade beeinträchtigt ist. Patienten mit von Willebrand-Syndrom (vWD) weisen daher oftmals Faktor VIII-Defizienz auf. Bei diesen Patienten ist die reduzierte Faktor VIII-Aktivität nicht die Konsequenz eines Defekts des X-chromosomalen Genes, sondern eine indirekte Folge der quantitativen und qualitativen Veränderung des vWF im Plasma. Die Unterscheidung zwischen Hämophilie A und vWD kann normalerweise durch Messen des vWF-Antigens oder durch Bestimmen der Ristocetin-Kofaktor-Aktivität erfolgen. Sowohl der vWF-Antigengehalt als auch die Ristocetin-Kofaktor-Aktivität ist bei den meisten vWD-Patienten erniedrigt, wogegen sie in Hämophilie A-Patienten normal ist.

Konventionelle Methoden zur Therapie des von Willebrand-Syndroms erfolgen mit aus Plasma gewonnenem vWF, wobei es eine Reihe von Ansätzen gibt, vWD-Patienten mit gereinigtem vWF oder Faktor VIII/vWF-Komplex zu therapieren. Die Entwicklung von monoklonalen und polyklonalen Antikörpern gegen Blutfaktoren, insbesondere gegen vWF, machte eine verbesserte Isolierung und Reinigung von vWF oder Faktor VIII/vWF-Komplex aus Plasma oder anderen biologischen Quellen möglich.

Die Reinigung von Faktor VIII bzw. Faktor VIII/vWF-Komplex aus Plasma wird insbesondere dadurch erschwert, daß Faktor VIII nur in sehr geringen Mengen im Plasma vorkommt, extrem labil ist, und die Assoziation von Faktor VIII mit vWF unter spezifischen Bedingungen reversibel ist.

Durch die Expression von Faktor VIII in rekombinanten Zellen (Wood et al., Nature 312: 330-337, 1984) konnte Faktor VIII gentechnisch hergestellt werden, jedoch konnte erst durch Zugabe oder Koexpression von vWF eine kommerziell einsetzbare Ausbeute an rekombinantem Faktor VIII erzielt werden. Ebenso wurde vWF durch gentechnische Verfahren hergestellt und in rekombinanten Zellen exprimiert (Fischer et al., 1994, FEBS Letters 351: 345-348).

Die Gewinnung von gereinigtem Faktor VIII, vWF oder Faktor VIII/vWF-Komplex aus Plasma oder Faktor VIII bzw. vWF aus rekombinanten Zellen mittels verschiedener Reinigungsverfahren wurde ebenfalls beschrieben.

Zimmerman et al. (US 4,361,509) beschreiben ein Verfahren zur Reinigung von Faktor VIII, wobei Faktor VIII/vWF-Komplex an einen monoklonalen anti-vWF-Antikörper gebunden wird und Faktor VIII vom Komplex durch CaCl₂ -Ionen dissoziiert wird. Der so erhaltene Faktor VIII wird anschließend über einen weiteren Chromatographieschritt in reiner Form gewonnen. Der Immunaffinitätsträger, an den vWF noch adsorbiert ist, wird mit einem chaotropen Agens, insbesondere NaSCN, regeneriert, wobei eine vWF/NaSCN-Lösung als Nebenprodukt anfällt und verworfen wird.

Die US 5,006,642 beschreibt die Gewinnung von vWF aus einer gemäß der US 4,361,509 als Nebenprodukt anfallenden Lösung aus vWF und chaotropem Agens durch Dialyse gegen einen geeigneten Puffer oder ein Entsalzen der Lösung durch einen weiteren chromatographischen Schritt.

Die EP 0 383 234 beschreibt die Herstellung eines vWF-Konzentrates mittels Anionenaustauschchromatographie, wobei ein in einer Lösung enthaltener Faktor VIII/vWF-Komplex durch Zugabe eines calcium- und aminosäurehaltigen Puffers dissoziiert wird und ein vWF-Konzentrat gewonnen wird.
Die EP 0 469 985 beschreibt ein Verfahren zur Herstellung von vWF aus Plasma-Kryopräzipitat, der weitgehend frei von Faktor VIII ist, bei dem in einem ersten Reinigungsschritt vWF vom Faktor VIII getrennt wird, wobei Faktor VIII an einen Anionenaustauscher bindet, wogegen vWF nicht gebunden wird. In einem zweiten Schritt wird die Salzkonzentration des vWF-haltigen Eluats verringert, wodurch vWF an einen zweiten Anionenaustauscher binden kann. vWF wird anschließend durch Erhöhung der Ionenstärke eluiert.

Für den Einsatz zur Therapie von Patienten mit von Willebrand-Syndrom, aber auch von Patienten mit phänotypischer Hämophilie A ist ein gereinigter vWF, der gegebenenfalls mit Faktor VIII komplexiert ist, wünschenswert. Zudem wird durch den stabilisierenden Effekt des vWF eine bessere Lagerstabilität von Faktor VIII-Präparaten erreicht.

Zur Reinigung des Faktor VIII/vWF-Komplexes wurde vorgeschlagen, kontaminierende Proteine, wie etwa Fibrinogen, mit hohen Konzentrationen an Aminosäuren, insbesondere von Glycin, auszufällen (WO 82/04395, EP 0 383 234).

Die EP 0 600 480 beschreibt die Reinigung von Faktor VIII/vWF-Komplex aus Plasma mittels einer Kombination aus Anionen/Kationen-Austauscherchromatographie, wobei der Faktor VIII/vWF-Komplex mit Heparin stabilisiert und gegebenenfalls Lysin als Antiprotease zugegeben wird.

Die EP 0 295 645 beschreibt die Reinigung von Faktor VIII/vWF-Komplex an einer Affinitätsmatrix, wobei als Affinitätsträger spezifische an vWF bindende Peptide eingesetzt werden, an die der Komplex bindet und anschließend mit einem pH-Gradienten eluiert wird.

Die WO 96/10584 beschreibt ein Verfahren zur Gewinnung von hochreinem rekombinanten vWF mittels kombinierter Anionenaustausch/Heparin-Affinitätschromatographie und die EP 0 705 846 die Trennung zwischen hoch-und niedermolekularen Fraktionen von vWF.

Mejan et al. (1988, Thromb. Haemost. 59: 364-371) schlugen vor, Faktor VIII/vWF-Komplex mittels Immunaffinität aus Plasma zu reinigen. Durch Verwendung eines vWF-spezifischen Antikörpers, gekoppelt an einen Träger, wird unter physiologischen Bedingungen Faktor VIII/vWF-Komplex an den Träger gebunden und unter alkalischen Bedingungen bei einem pH-Wert von 10,2 der Komplex eluiert. Das Eluat wird sofort zur Neutralisation des alkalischen Milieus mit 2 M Essigsäure versetzt, mit humanem Serumalbumin stabilisiert und anschließend lyophilisiert. Das schwach alkalische Elutionsmilieu wurde insbesondere deshalb ausgewählt, um eine Dissoziation des Faktor VIII/vWF-Komplexes und eine Inaktivierung von Faktor VIII zu verhindern.

Es wird jedoch immer wieder betont, daß eine besondere Schwierigkeit bei der Reinigung des Komplexes darin besteht, die Assoziation der Proteine zu erhalten, da der Komplex der beiden Komponenten instabil ist. Mejan et al. (1988, Thromb. Haemost. 59: 364-371) erreichten mit ihrem Verfahren eine 50%ige Zurückgewinnung des Faktor VIII/vWF-Komplexes mit einer spezifischen Aktivität von Faktor VIII ≥ 20 U/mg und von vWF ≥ 20 U/mg. Unter den beschriebenen Elutionsbedingungen wurde jedoch eine teilweise Freisetzung der Antikörper von der Säule beobachtet, was zu einer Kontamination des Eluats mit etwa 90 ng/ml Eluat an Maus-IgG führte. Die Antikörper mußten daher durch einen weiteren chromatographischen Schritt entfernt werden.

Hornsey et al. (1987, Thromb. Haemost. 57: 102-105) untersuchten den Einfluß verschiedener Elutionsmittel bei der Immunaffinitätsreinigung von Faktor VIII/vWF-Komplex und stellten fest, daß chaotrope Agentien, wie Kaliumiodid und Lithiumdiiodsalicylate besonders effektiv als Elutionsmittel wirken. Das verwendete Elutionsmittel enthielt zudem Calciumchlorid-Ionen sowie zur Stabilisierung der Faktor VIII- und vWF-Aktivität 1 M Lysin. Es wurde beobachtet, daß die hohe Konzentration der Aminosäure die Proteine vor dem denaturierenden Effekt des chaotropen Agens schützt. Unter den verwendeten Elutionsbedingungen wurde gefunden, daß das Endprodukt mit dem Immunadsorbens, hier Maus-monoklonale vWF-Antikörper, bis zu 30 ng/ml (etwa 300 ng/mg Protein) verunreinigt war. Das chaotrope Desorptionsmittel mußte ebenfalls aufgrund seiner hohen Toxizität aus dem Produkt durch einen zusätzlichen Reinigungsschritt entfernt werden. Hornsey et al. (1987, Thromb. Haemost. 57: 102-105) erreichten durch die Immunaffinitätschromatographie eine Ausbeute von 57% Faktor VIII:C und 44% vWF und eine spezifische Aktivität von 45 U Faktor VIII und 60 U Ristocetin-Aktivität/mg Protein.

Obwohl für die Reinigung von vWF oder Faktor VIII/vWF-Komplex die Immunaffinität eine der bevorzugten Methoden ist, ist der größte Nachteil bei der Verwendung der Immunaffinitätschromatographie, die mögliche Kontamination des Endproduktes mit dem Immunadsorbens sowie die notwendige Regenerierung der verwendeten Affinitätsmatrix. Die starke Bindung zwischen Antikörper und vWF bzw. Faktor VIII/vWF-Komplex macht es oft notwendig, ein starkes Desorptionsmittel, wie etwa ein chaotropes Agens, zu verwenden. Dadurch wird nicht nur die Aktivität und molekulare Struktur des vWF oder des Faktor VIII/vWF-Komplexes beeinträchtigt, sondern hat auch durch ein kontinuierliches Freisetzen von Antikörpern vom Träger ein Auslaugen ("leakage") des Immunträgers zur Folge. Die Halbwertszeit des Affinitätsträgers wird stark herabgesetzt und seine mehrfache Verwendbarkeit ist beeinträchtigt. Da insbesondere für die kommerzielle Präparation von vWF oder Faktor VIII/vWF-Komplex die Verwendung von Immunaffinitätssäulen sehr kostspielig ist, wäre es daher wünschenswert, ein Verfahren zur Verfügung zu stellen, das diese Nachteile nicht aufweist.

Das Ziel der vorliegenden Erfindung ist es daher, ein verbessertes Verfahren zur Gewinnung von vWF bzw. Faktor VIII/vWF-Komplex mittels Immunaffinitätschromatographie, das die oben beschriebenen Nachteile nicht aufweist, zur Verfügung zu stellen.

Die Aufgabe wird erfindungsgemäß dadurch gelöst, daß ein Verfahren zur Verfügung gestellt wird, bei dem vWF oder Faktor VIII/vWF-Komplex mittels Immunaffinitätschromatographie gereinigt wird, wobei ein an ein Immunadsorbens gebundener vWF oder Faktor VIII/vWF-Komplex mit einem Elutionsmittel, enthaltend als wesentlichen aktiven Bestandteil ein Zwitterion, eluiert wird und wobei der an Immunadsorbens gebundene vWF bzw. Faktor VII/vWF-Komplex ohne die Verwendung von chaotropen Reagentien dissoziiert wird.

Als Zwitterionen im Elutionsmittel gemäß der vorliegenden Erfindung werden Aminosäuren oder Analoge von Aminosäuren verwendet, deren isoelektrischer Bereich zwischen 3,2 und 9,6 liegt und die im neutralen pH-Bereich als Zwitterion vorliegen. Die bevorzugten Zwitterionen sind dabei ausgewählt aus der Gruppe von neutralen Aminosäuren oder Analogen von Aminosäuren, vorzugsweise Alanin, β-Alanin, 2-Aminobuttersäure, 4-Aminobuttersäure, Asparagin, Citrullin, Glutamin, Glycin, Histidin, Isoleucin, Leucin, Methionin, Phenylalanin, Prolin, Sarkosin, Serin, Taurin, Threonin, Tryptophan und Tyrosin, oder Betaine oder Analoge davon, insbesondere Sulfobetaine.

Besonders bevorzugt sind zur Durchführung des Verfahrens die Aminosäuren Alanin, β-Alanin, Glycin, Histidin, Phenylalanin, oder Betain. Die Zwitterionen können gemäß dem vorliegenden Vefahren in einer wässrigen Lösung in einer Konzentration zwischen 0,01 und 0,5 M, vorzugsweise zwischen 0,08 und 0,2 M, besonders bevorzugt zwischen 0,1 und 0,15 M vorliegen. Wässrige Lösungen können dabei Lösungen sein, die ausschließlich aus Wasser bestehen und keine weiteren Zusätze enthalten.

Das Elutionsmittel enthaltend das Zwitterion kann gemäß dem vorliegenden Verfahren einen physiologischen pH-Wert aufweisen, vorzugsweise einen pH-Wert zwischen 6,5 und 8,0, besonders bevorzugt zwischen 7,0 und 7,8, insbesondere bevorzugt von 7,4. In diesem pH-Bereich verhalten sich alle oben genannten Aminosäuren oder Analoge davon elektrisch neutral und liegen als Zwitterionen vor.

Das Elutionsmittel kann durch einfaches Lösen der Aminosäure oder des Analogen einer Aminosäure mit der gewünschten Molarität in Wasser hergestellt werden. Da durch die neutrale Ladung des Zwitterions sich der pH-Wert und die Ionenstärke nicht oder nur unwesentlich ändern, ist ein Einstellen eines pH-Wertes oder der Ionenstärke nicht unbedingt notwending. Da in einigen Fällen, beispielsweise wenn ein oder mehrere Virusinaktivierungsschritte durchgeführt werden, eine zusätzliche Stabilisierung des Eluats enthaltend den vWF bzw. vWF-Komplex wünschenswert ist, können gegebenenfalls als weitere Substanzen Zucker, etwa Saccharose oder Maltose, als Stabilisatoren zugesetzt werden. Da sich diese Zucker ebenfalls chemisch/physikalisch neutral verhalten, erfolgt bei Zugabe keine pH-Änderung des Elutionsmittels.

Die Verwendung von Aminosäuren, wie etwa Glycin, Histidin oder Arginin als Fällungsmittel, Antiprotease oder Stabilisator, oder zur verbesserten Rekonstitution von Faktor VIII-Lösungen wurde z.B. in der EP 0 383 234 oder der EP 0 600 480 beschrieben, wobei die Konzentration der eingesetzten Aminosäuren zwischen 0,5 - 3 M lag. Zudem sind Aminosäuren als Bestandteil von Elutionsmittel oder Puffern bei chromatographischen Verfahren bekannt. Tsang et al. (1991, J. Immunol. Methods 138: 291-299) untersuchten verschiedene Dissoziationsbedingungen von an Immunaffinitätsmatrices gebundenen Antigenen und fanden, daß chaotrope Mittel die effektivsten Dissoziationsreagentien zur Lösung von Antigen-Antikörper-Bindungen sind. Die spezifische Aktivität der eluierten Proteine war jedoch aufgrund der denaturierenden Wirkung der chaotropen Agentien gering. Die Verwendung von 0,5 M Glycin, pH 2,0, als Elutionreagens zeigte eine vergleichsweise geringe Dissoziationskapazität und eine relativ geringe spezifische Aktivität des gewonnenen Proteins. Der niedrige pH-Wert des Elutionsmittels machte es zudem notwendig das Eluat schnell zu neutralisieren, um einen weiteren Aktivitätsverlust des Proteins zu vermeiden. Ebenso wurde gefunden, daß sich unter diesen Bedingungen Antikörper vom Träger lösen und ins Eluat gelangen.

Aufgrund der Beobachtungen von Tsang et al. (1991, J. Immunol. Methods 138: 291-299) war es daher um so überraschender, daß im Rahmen der vorliegenden Erfindung gefunden wurde, daß ein Elutionsmittel enthaltend als wesentliches aktives Elutionsreagens ein Zwitterion, in der Lage ist, einen Antikörper/Antigen-Komplex unter milden Bedingungen und im neutralen pH-Bereich und ohne Verwendung von chaotropen Agentien effektiv zu dissoziieren. Durch die milden Elutionsbedingungen wird nicht nur effizient das gewünschte Antigen in gereinigter Form, und aufgrund der hohen Spezifität zum Antikörper, frei von verunreinigenden Bestandteilen erhalten, sondern kann auch im wesentlichen frei von Immunadsorbens gewonnen werden, da zwar eine Dissoziation des Antikörper-Antigen-Komplexes erfolgt, die Bindung des Antikörpers an den Träger jedoch nicht beeinflußt wird und der Antikörper stabil mit dem Träger assoziiert bleibt.

Zur Durchführung des erfindungsgemäßen Verfahrens kann als Ligand jedes Immunadsorbens, das eine Bindungsspezifität zu vWF besitzt und an einem festen Träger als Ligand immobilisiert werden kann, verwendet werden.

Gemäß einer Ausführungsform des erfindungsgemäßen Verfahrens wird ein anti-vWF-Antikörper als Immunadsorbens verwendet. Der eingesetzte Antikörper kann polyklonal oder monoklonal sein. Besonders bevorzugt sind jedoch monoklonale Antikörper. Zur Durchführung des erfindungsgemäßen Verfahrens können alle bekannten anti-vWF-Antikörper eingesetzt werden, etwa solche wie sie von Goodall et al. (Thromb. Haemost. 54: 878-891, 1985) beschrieben sind. Vorzugsweise wird ein monoklonaler vWF-Antikörper aus einem Hybridom, ausgewählt aus der Serie von Zellhybridisierungen von Mäusemyelom X63 und Splenozyten von BALB/C-Maus, immunisiert mit Faktor VIII/vWF-Komplex, wie sie etwa in der EP 0 179 006 beschrieben werden, eingesetzt.

Gemäß einer bevorzugten Ausführungsform wird zur Durchführung des erfindungsgemäßen Verfahrens als Ligand ein Fragment eines anti-vWF-Antikörpers, der vWF bzw. Faktor VIII/vWF-Komplex bindet, eingesetzt. Besonders bevorzugt ist dabei das F'ab-Fragment eines vWF-Antikörpers. Die Verwendung eines Fragmentes eines spezifischen Antikörpers hat den Vorteil, daß auf einfache Weise eine Affinitätsträgermatrix mit dem Antikörper-Fragment als Liganden hergestellt werden kann, wobei durch die geringere molekulare Größe des Liganden die Antigenbindungskapazität und Bindungseffizienz des Trägers erhöht werden kann. Auch wird das Risiko, daß durch einen möglicherweise ins Eluat gelangenden kompletten Antikörper, das Produkt verunreinigt wird, stark herabgesetzt und damit vernachlässigbar.

Das Immunadsorbens ist vorzugsweise an einen Träger immobilisiert, wobei als Träger alle bekannten natürlichen oder synthetischen unlöslichen Polymere, die für die Affinitätschromatographie geeignet sind, verwendet werden können.
Das erfindungsgemäße Verfahren stellt eine einfache und effiziente Methode zur Gewinnung von vWF oder Faktor VIII/vWF-Komplex mit einer hohen spezifischen Aktivität und Ausbeute aus einer gegebenenfalls nicht vorgereinigten Proteinlösung dar. Als Ausgangsmaterial enthaltend vWF oder Faktor VIII/vWF-Komplex kann dabei jede biologische Lösung, etwa Plasma, ein Plasmapool, eine Plasmafraktion, ein Kryopräzipitat, der Überstand einer Zellkultur, die rekombinanten vWF oder Faktor VIII exprimiert bzw. Faktor VIII und vWF co-exprimiert, oder eine vorgereinigte Proteinlösung dienen. Das vorliegende Verfahren ist jedoch besonders geeignet, aus nicht-vorgereinigten Proteinlösungen, etwa direkt aus Plasma oder dem Überstand einer rekombinanten Zellkultur, vWF oder Faktor VIII/vWF-Komplex zu gewinnen. vWF oder Faktor VIII/vWF-Komplex enthalten in einem Ausgangsmaterial wird gemäß dem vorliegenden Verfahren vorzugsweise unter physiologischen Bedingungen an das Immunadsorbens gebunden. Dabei wird die Ausgangslösung bei einer Temperatur zwischen 4°C und 20°C auf einen pH-Wert zwischen 6,5 und 7,8 eingestellt und mit dem Immunadsorbens in Kontakt gebracht, wodurch aufgrund der spezifischen Bindungsfähigkeit des Antikörpers zum vWF, der vWF bzw. Faktor VIII komplexiert mit vWF gebunden wird, wogegen andere, in der Lösung enthaltene Proteine nicht gebunden und so in einfacher Weise vom vWF oder Faktor VIII/vWF-Komplex abgetrennt werden.

Durch das erfindungsgemäße Verfahren und Elution mit einem Puffer im neutralen pH-Bereich, enthaltend als wesentliches, eluierendes Agens ein Zwitterion, lassen sich vWF oder Faktor VIII/vWF-Komplex vom Immunadsorbens mit besonders hoher Effizienz gewinnen. Durch die spezifische Interaktion von vWF und Faktor VIII in einem Komplex wird auch Faktor VIII komplexiert mit vWF gebunden, wogegen freier, nicht-komplexierter Faktor VIII nicht an das Immunadsorbens binden kann. Mit Hilfe des erfindungsgemäßen Verfahrens wird daher reiner vWF bzw. ausschließlich mit vWF komplexierter Faktor VIII gewonnen. Insbesondere wurde festgestellt, daß etwa 85% des im Ausgangsmaterial vorhandenen vWF bzw. Faktor VIII/vWF-Komplexes zurückgewonnen werden können. Durch das erfindungsgemäße Verfahren wird daher vWF bzw. Faktor VIII/vWF-Komplex mit einer Reinheit von mehr als 90%, vorzugsweise von mehr als 95% wiedergewonnen. Diese hohe Wiedergewinnungsrate und Reinheit wurde mit bisher beschriebenen Chromatographieverfahren in einem Verfahrensschritt nicht erreicht. Auch haben die Verwendung des beschriebenen Elutionsmittels und die milden Elutionsbedingungen den Vorteil, daß die Assoziation des Faktor VIII mit vWF und damit die physiologische und molekulare Struktur des Komplexes erhalten bleibt.

Das erfindungsgemäße Verfahren hat als weiteren besonderen Vorteil, daß vor und während der Elution keine pH-Änderung der Lösung enthaltend die Proteine erfolgen muß und die biologische Aktivität des zu reinigenden Proteins durch eine pH-Änderung nicht beeinträchtigt wird.

Durch die Verwendung des oben beschriebenen Elutionsmittels wird der vWF bzw. der vWF-Komplex effizient vom Immunadsorbens desorbiert, ohne die strukturelle Integrität des vWF bzw. die Aktivität des vWF oder Faktor VIII zu beeinträchtigen. Die Unversehrtheit der strukturellen Integrität des vWF wurde insbesondere durch vWF-Multimeranalyse, wie etwa in der EP 0 705 846 beschrieben, vor Adsorption und nach Elution vom Immun-Träger nachgewiesen. Ebenso hat sich gezeigt, daß die Kollagenbindungsaktivität, sowie die spezifische Plättchenagglutinationsaktivität des vWF im wesentlichen nicht beeinträchtigt ist.

Ein Vergleich der Faktor VIII : Ag zur Faktor VIII:C-Aktivität im Ausgangsmaterial und nach Elution vom Träger zeigte, daß das Verhältnis sich nicht verändert. Die spezifische Aktivität beträgt vorzugsweise > 80 U/mg.

Der mit dem erfindungsgemäßen Verfahren erhaltene hochreine vWF bzw. Faktor VIII/vWF-Komplex kann gegebenenfalls durch aus dem Stand der Technik bekannte chromatographische Methoden, wie Ionenaustausch-, Affinitäts- oder Gelfiltrationschromatographie weiter gereinigt werden. Gereinigter Faktor VIII/vWF-Komplex kann beispielsweise an einen Affinitätsträger, der entweder vWF oder Faktor VIII spezifisch bindet, adsorbiert werden, und die Proteine mit einem Puffer, der den Komplex dissoziiert, etwa einem Puffer mit niedrigem pH-Wert, hoher Ionenstärke, enthaltend CaCl₂ oder NaCl, getrennt voneinander und in freier Form gewonnen werden. Durch diese Vorgangsweise kann selektiv freier, nicht mit vWF-komplexierter Faktor VIII bzw. nicht mit Faktor VIII-komplexierter vWF gewonnen werden. Die so isolierten Proteine oder Antigene zeichnen sich insbesondere dadurch aus, daß sie in bezug auf die vWF-Bindung bzw. Faktor VIII-Bindung nicht beeinträchtigt sind. Dadurch kann gezielt hochreiner Faktor VIII mit einer hohen vWF-Bindungsspezifität bzw. hochreiner vWF mit hoher Faktor VIII-Bindungsspezifität im Verhältnis zum Antigengehalt gewonnen werden.

Die vorliegende Erfindung umfaßt daher auch hochreinen vWF mit einer Faktor VIII-Bindungskapazität von mehr als 90%, vorzugsweise mehr als 95% bzw. Faktor VIII mit einer vWF-Bindungsaffinität von mindestens 90%. Ein derart gereinigter vWF bzw. Faktor VIII/vWF-Komplex kann etwa wie in der EP 0 705 846 beschrieben mittels Heparinaffinitätschromatographie weitergereinigt werden, wodurch hochmolekulare vWF-Multimere angereichert werden. Der so erhaltene vWF bzw. Faktor VIII/vWF-Komplex enthaltend insbesondere hochmolekulare vWF-Multimere zeichnet sich durch eine hohe spezifische Plättchenaggregation von mindestens 60 U/mg Protein aus.

Insbesondere wird mit dem erfindungsgemäßen Verfahren gereinigter vWF bzw. Faktor VIII/vWF-Komplex in einer wässrigen Lösung, enthaltend ein Zwitterion in einer Konzentration zwischen 0,08 M bis 0,2 M und einem pH zwischen 7,3 und 7,8, gewonnen, wobei das Zwitterion ausgewählt ist aus der Gruppe der Aminosäuren Glycin, Alanin, β-Alanin, Phenylalanin und Histidin, oder der Betaine. Dieser gereinigte vWF bzw. Faktor VIII/vWF-Komplex kann dann gegebenenfalls einem weiteren aus dem Stand der Technik bekannten Virusabreicherungs- und/oder Virusinaktivierungsschritt unterzogen werden.

Die gereinigte Lösung kann beispielsweise direkt über einen Filter mit einer Porenweite zwischen 100 nm bis 0,45 µm sterilfiltriert und ohne Zusatz eines Stabilisators, insbesondere eines hochmolekularen Stabilisators, lyophilisiert werden.

Das erfindungsgemäße Verfahren kann zur Reinigung und Gewinnung von rekombinantem oder plasmatischem vWF bzw. Faktor VIII/vWF-Komplex eingesetzt werden.

Gemäß der vorliegenden Erfindung enthält die stabile Präparation vWF mit einer Reinheit von mindestens 95% und einer spezifischen Antigen-Aktivität von mindestens 95 U/mg Protein, vorzugsweise von 100 U/mg Protein. Eine erfindungsgemäß bereitgestellte stabile Präparation enthaltend Faktor VIII/vWF-Komplex weist eine Reinheit von mindestens 95%, vorzugsweise von 99%, wobei die spezifische Aktivität von Faktor VIII:C mindestens 95 U/mg Protein und von vWF von mindestens 95 U/mg Protein aufweist und damit das Verhältnis der spezifischen Aktivität im Komplex 1:1 ist.

Gemäß einer besonderen Ausführungsform besteht der Faktor VIII/vWF-Komplex aus rekombinantem Faktor VIII und rekombinantem vWF, wobei das Verhältnis von Faktor VIII zu vWF zwischen 0,01 und 100, vorzugsweise zwischen 1:30 und 1:70, besonders bevorzugt bei ungefähr 1:50 liegt. Es wurde im Rahmen der Erfindung gefunden, dass sich die Zellkulturüberstände von rekombinanten vWF- bzw. Faktor VIII-exprimierenden Zellen mit einer definierten Konzentration an Antigen in einem bestimmten beliebigen Verhältnis mischen lassen und nach Durchführung des erfindungsgemäßen Verfahrens gereinigter Komplex bestehend aus rekombinanten Proteinen auch in dem gewünschten Verhältnis wiedergewinnen lässt.

Bekanntermaßen wird vWF bei der Reinigung aus Plasma oder Kryopräzipitat durch gegebenenfalls anwesende Proteasen zu niedermolekularen vWF-Fragmenten degradiert, die dann als niedermolekulare Fraktion mit Satellitenbanden im SDS-Gel identifiziert werden können (Furlan et al., 1993, PNAS. 90: 7503-7507). Auch wird Faktor VIII durch anwesende Proteasen teilweise aktiviert, wodurch die spezifische Aktivität des Faktor VIII/vWF-Komplexes reduziert wird.

Die erfindungsgemäß hergestellte stabile Präparation enthaltend hochreinen vWF bzw. Faktor VIII/vWF-Komplex zeichnet sich insbesondere dadurch aus, dass die molekulare und strukturelle Integrität des vWF bzw. des Faktor VIII/vWF-Komplexes im Wesentlichen erhalten ist und dass sie keine proteolytischen Abbauprodukte des vWF oder aktivierten Faktor VIII enthält.

Die erfindungsgemäß hergestellte Präparation ist im Wesentlichen frei von durch das Immunadsorbens verursachte Verunreinigungen, wobei die Menge an gegebenenfalls vorhandenem Immunadsorbens, insbesondere an Antikörper, weniger als 15 ng/mg Protein, vorzugsweise weniger als 10 ng/mg Protein und besonders bevorzugt weniger als 7 ng/mg Protein beträgt. Desweiteren zeichnet sich die erfindungsgemäße Präparation dadurch aus, dass sie weniger als 3%, vorzugsweise weniger als 1% Fibrinogen und weniger als 1%, vorzugsweise weniger als 0,5% Fibronektin-Gehaltes erfolgte dabei mittels ELISA gemäß dem Stand der Technik.

Im Rahmen der vorliegenden Erfindung hat sich gezeigt, dass insbesondere durch die Verwendung eines Elutionsmittels enthaltend als wesentliches eluierendes Agens im Puffer eine Aminosäure, wie Glycin, Histidin oder Alanin, die bisher auch zur Stabilisierung von Proteinlösungen eingesetzt wurden, der eluierte vWF oder Faktor VIII/vWF-Komplex während des gesamten Reinigungsverfahrens in einem stabilisierenden Milieu gehalten werden kann und daher die gewonnene Präparation besonders stabil ist.

Die erfindungsgemäß hergestellte Präparation ist in Lösung bei 4°C über einen Zeitraum von mindestens 2 Wochen, bei -20°C tiefgefroren für mindestens 6 Monate und als Lyophilisat mindestens 1 Jahr bei einer Temperatur von -20°C bis 4°C stabil. Durch die Anwesenheit etwa von Histidin im Elutionsmittel wird zudem eine Präzipitation des vWF- bzw. Faktor VIII/vWF-Komplexes beim Lyophilisiervorgang verhindert. Die erfindungsgemäße Präparation kann daher gegebenenfalls ohne weitere Zusätze direkt zu einem lyophilisierten Produkt weiterverarbeitet werden. Zur Verbesserung der Rekonstitution des lyophilisierten Präparates kann auch, wie etwa in der WO 93/22336 beschrieben, Arginin zugesetzt werden. Die stabile Präparation kann zur Verabreichung am Menschen in entsprechender Weise als stabile pharmazeutische Präparation formuliert werden und geeignete Puffer oder physiologisch akzeptable Träger enthalten. Wie oben schon erwähnt, kann aufgrund der besonders guten Stabilität der Präparation gegebenenfalls auf den Zusatz eines Stabilisators verzichtet werden. Bekanntermaßen wird durch die Zugabe eines Stabilisators, insbesondere von HSA, die spezifische Aktivität einer Antigenpräparation reduziert, wodurch die verabreichbare pharmazeutische Zusammensetzung eine geringere spezifische Aktivität aufweist als die direkt nach der Reinigung gewonnene Proteinpräparation.

Gemäß der vorliegenden Erfindung wird daher eine pharmazeutische Zusammensetzung enthaltend vWF bzw. Faktor VIII/vWF-Komplex mit einer Reinheit von mindestens 95%, vorzugsweise 99%, und einer spezifischen Aktivität von vWF von mindestens 95 U/mg und Faktor VIII von mindestens 95 U/mg bereitgestellt, die im wesentlichen frei ist von hochmolekularen Substanzen und Stabilisatoren, insbesondere von HSA. Dadurch unterscheidet sich die erfindungsgemäße pharmazeutische Präparation von bisher bekannten pharmazeutischen Zusammensetzungen, die zur Stabilisierung Substanzen wie etwa Albumin oder Heparin enthalten.

Prinzipiell bedarf es jedoch keiner weiteren aufwendigen Umformulierung der gereinigten Präparation zur Verabreichung am Menschen, da das gereinigte Produkt schon in einer physiologisch verträglichen Zusammensetzung vom Affinitätsträger gewonnen werden kann. Ebenso liegt es bereits im Eluat in einer stabilen und stabilisierten Form vor.

Das erfindungsgemäße Verfahren stellt somit ein einfaches Verfahren zur schonenden Gewinnung von hochreinem vWF bzw. Faktor VIII/vWF-Komplex und der einfachen Herstellung eines stabilen pharmazeutischen Präparates dar, das gegebenenfalls direkt für eine therapeutische Anwendung verwendet werden kann.

Vor der Verarbeitung des hochreinen vWF bzw. vWF-Komplexes zu einer pharmazeutischen Präparation ist es von Vorteil, eine Inaktivierung oder Abreicherung von Viren vorzunehmen. Dies kann durch im Stand der Technik bekannte chemische und/oder physikalische Behandlung erfolgen. Da durch die Anwesenheit von Aminosäuren, wie Glycin, Alanin oder Histidin, enthalten in der erfindungsgemäßen stabilen Präparation, vWF bzw. Faktor VIII/vWF-Komplex in einem physiologischen und stabilisierenden Milieu vorliegt, kann das gereinigte Produkt gegebenenfalls ohne weitere Zusätze von Stabilisatoren direkt einem Virusinaktivierungsschritt unterzogen werden.

Die gemäß der vorliegenden Erfindung zur Verfügung gestellte stabile Präpration kann zur Herstellung eines Arzneimittels zur Behandlung von Hämophilie A oder der vWD verwendet werden.

Die Erfindung wird anhand der nachfolgenden Beispiele und der Zeichnungsfigur näher erläutert, wobei sie jedoch nicht auf diese besonderen Ausführungsformen eingeschränkt ist.

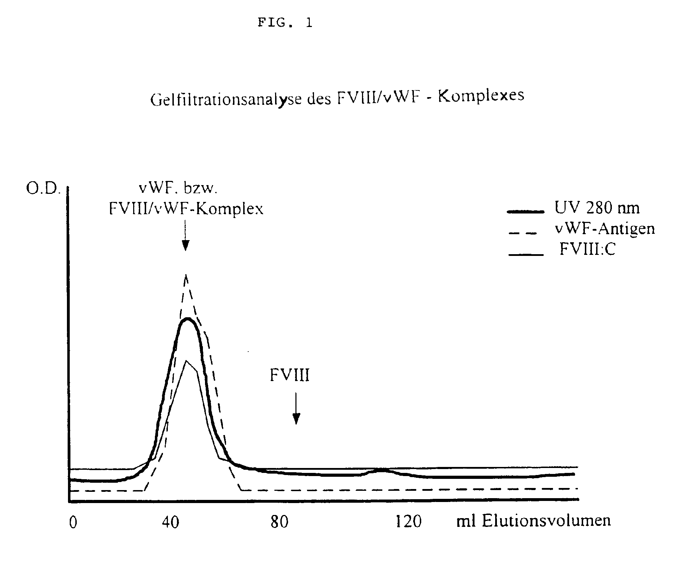

Es zeigt: Fig. 1: eine Gelfiltrationsanalyse des Faktor VIII/vwF-Komplexes.

Beispiel 1 beschreibt die Bestimmung der Wechselwirkung von verschiedenen anti-vWF-Antikörpern und vWF mittels "Surface Plasmon Resonance" und Desorption des Antigens vom Komplex mit verschiedenen Puffern; Beispiel 2 beschreibt das Desorptionsverhalten des vWF-Antigens vom Antigen-Antikörper-Komplex unter verschiedenen Pufferbedingungen; Beispiel 3 beschreibt die Reinigung von rekombinantem vWF aus Zellkulturüberständen mittels Immunaffinität; Beispiel 4 beschreibt die Reinigung von plasmatischem Faktor VIII/vWF-Komplex; Beispiel 5 beschreibt die Verwendung eines Antikörperfragmentes für die Immunaffinitätsreinigung von vWF; Beispiel 6 beschreibt die Nachreinigung von immunaffinitätsgereinigtem vWF mittels Heparin-Affinitätschromatographie; Beispiel 7 beschreibt den Einfluß des Elutionsmittels auf die Faktor VIII:C-Aktivität; Beispiel 8 beschreibt die Reinigung eines Komplexes bestehend aus rekombinantem Faktor VIII und rekombinantem vWF; Beispiel 9 beschreibt die Austestung des Eluates auf Maus-IgG und F'ab-Fragmente und Beispiel 10 zeigt die Integrität des Faktor VIII/vWF-Komplexes im erfindungsgemäßen Elutionsmilieu.

### Beispiele

### Allgemeine Methodenbeschreibung zum Testsystem zur Bestimmung der Wechselwirkung von Makromolekülen

Die Wechselwirkung von Makromolekülen, dazu zählt auch die Reaktion von Proteinen mit spezifischen Antikörpern, kann seit kurzer Zeit mit Hilfe einer neuen Technologie - der Surface Plasmon Resonance - direkt verfolgt werden (Karlsson, 1994, Analyt. Biochem. 221: 142-151; Malmqvist, 1993, Nature 361: 186-187).

In einem optischen System, welches in direktem Kontakt mit der zu untersuchenden Probe steht, wird ein von der Molekül-Konzentration abhängiger Parameter gemessen und in sogenannten "Response-Units (RU)" wiedergegeben. Hohe RU-Werte entsprechen einer hohen Konzentration von adsorbierten Makromolekülen an der Meßoberfläche (Sensor Chip).

Die Vorteile der Methode bestehen im geringen Substanzverbrauch, der schnellen Durchführbarkeit und der Möglichkeit der kinetischen Auswertung der Daten.
Mit Hilfe dieses Meßsystems wurden die grundlegenden Bindungseigenschaften von vWF-spezifischen Antikörpern untersucht.

### Beispiel 1:

### Bestimmung der Wechselwirkung von verschiedenen anti-vWF-Antikörpern und vWF mittels "Surface Plasmon Resonance" und Desorption des Antigens vom Komplex mit verschiedenen Puffern

Monoklonale anti-vWF-Antikörper (Immunotech, Marseille) wurden mittels der von Pharmacia angegebenen Standardmethode an die aktive Schicht eines CM-Sensorchips gebunden. Als Negativkontrolle wurde eine Spur des Sensorchips mit einem monoklonalen Antikörper gegen Pseudomonas Flagellin (PAM 24) beschichtet. Die Effektivität der Kopplung ist aus dem Anstieg des Signals zu erkennen. Die Spuren des Sensorchips wurden nach der Kopplung mit 3M NaSCN gewaschen, um unspezifisch adsorbierten Antikörper von der Chipoberfläche zu entfernen. vWF (vorgereinigt über Anionentauscher-chromatographie) wurde anschließend über die Chipoberfläche geleitet (Adsorptionsphase). Nach einem Waschvorgang mit Tris-Puffer, pH 7,4, wurden verschiedene Puffer enthaltend 100 mM Glycin und steigenden pH-Werten von pH 6,0 bis pH 9,0 über den Chip geleitet (Elutionsphase). Tabelle 1 zeigt die Bestimmung der Wechselwirkung von veschiedenen anti-vWF-Antikörpern und vWF mittels "Surface Plasmon Resonance" und Desorption des Antigens vom Komplex mit Glycin-Puffer mit verschiedenen pH-Werten.

**Tabelle 1**

| | | | Glycin-Elution | | | | | | | |
|---|---|---|---|---|---|---|---|---|---|---|
| Spur | vWf Beladung (RU) | | pH 6 | | pH 7 | | pH 8 | | pH 9 | |
| | r-vWF | pdvWF | r-vWF | pdvWF | r-vWF | pdvWF | r-vWF | pdvWF | r-vWF | pdvWF |
| AvW8-1 | 650 | 490 | 145 | 220 | 40 | 65 | 15 | 20 | 10 | 20 |
| AvW8-2 | 870 | 760 | 75 | 125 | 25 | 50 | 10 | 15 | 15 | 12 |
| PAM 24 | 12 | 25 | 10 | 15 | 10 | 15 | 10 | 15 | 10 | 15 |
| Leerspur | 14 | 35 | -5 | 5 | -20 | 5 | -20 | 5 | -20 | 5 |

Laut Offenbarung von Mejan et al. (1988, Thromb. Haemost. 59: 364-371) liegt die maximale Affinität zwischen Antikörper und Antigen zwischen pH 5 und 7. Es war daher nicht zu erwarten, daß schon ab einem pH -Wert von 6,0 eine deutliche Ablösung des adsorbierten r-vWF erfolgte. Die Änderung des Desorptionsverhaltens des Antigen-Antikörper-Komplexes wurde auf Anwesenheit von Glycin im Puffer zurückgeführt. Weiters zeigte sich, daß die beiden unterschiedlichen anti-vWF-Antikörper AvW8-1 und AvW8-2 das gleiche Verhalten gegenüber Glycin aufweisen.

Um auszuschließen, daß es sich bei dem beobachteten Effekt um eine spezielle Eigenschaft des rekombinanten vWF handelt - wurde das Experiment, mit gleichem Ergebnis, mit plasmatischem vWF (pdvWF) wiederholt.

### Beispiel 2:

### Desorptionsverhalten des vWF-Antigens vom Antigen-Antikörper-Komplex unter verschiedenen Pufferbedingungen

Um die Natur der vWF-Antigen/anti-vWF-Antikörper-Wechselwirkung näher zu untersuchen, wurden weitere Elutionsversuche mit anderen Puffern/Elutionsmitteln durchgeführt. Vor allem wurde die Wirksamkeit von Puffern enthaltend verschiedene Aminosäuren bzw. deren Derivate bei pH 7,4 untersucht.

Zu den untersuchten Substanzen gehörten u.a.: Alanin, b-Alanin, Phenylalanin, Histidin, Arginin, Lysin, Glutaminsäure, Asparaginsäure, Betain und Acetat.

Tabelle 2 zeigt die Bestimmung der Wechselwirkung von anti-vWF-Antikörpern und vWF-Antigen mittels "Surface Plasmon Resonance" und Desorption des Antigens vom Komplex mit Puffern enthaltend verschiedene Aminosäuren als Elutionsreagens.

**Tabelle 2**

| Aminosäure | RU vor Elution | RU nach Elution | % eluiert |
|---|---|---|---|
| Alanin | 850 | 25 | 97 |
| β-Alanin | 830 | 30 | 97 |
| Phenylalanin | 520 | 25 | 95 |
| Histidin | 550 | 15 | 97 |
| Arginin | 970 | 780 | 20 |
| Lysin | 840 | 730 | 13 |
| Glutaminsäure | 760 | 720 | 5 |
| Asparaginsäure | 670 | 590 | 12 |
| Betain | 680 | -10 | 100 |
| Acetat | 570 | 550 | 3 |

Gruppen wie Glycin, Alanin, β-Alanin oder Phenylalanin oder Derivate davon bzw. Aminosäuren, welche im neutralen Bereich als Zwitterionen vorliegen, wie Histidin, in der Lage sind, den an den Antikörper gebundenen vWF effektiv zu eluieren (Tabelle 2).

Entsprechende Puffer enthaltend diese Aminosäuren bzw. Derivate davon wurden für die Reinigung von vWF bzw. vWF/Faktor VIII-Komplex mittels Immunaffinitätschromatographie getestet.

### Beispiel 3:

### Reinigung von rekombinantem vWF aus Zellkulturüberständen mittels Immunaffinität (derzeit nach Ansicht der Anmelderin der beste Weg zur Ausführung der Erfindung)

Der monoklonale anti-vWF-Antikörper AvW8-2 wurde gemäß Instruktionen des Herstellers kovalent an CNBr-aktivierte Sepharose (Pharmacia) gekoppelt. Die Belegung des Harzes mit Antikörper betrug dabei 1 mg/ml gepacktes Gel.

500 ml eines konzentrierten Fermentationsüberstandes enthaltend rekombinanten vWF (rvWF) wurden mit einer linearen Flußrate von 20 cm/h auf einer Säule, gefüllt mit 50 ml Affinitätsharz, aufgetragen. Nach dem Auftragen der Probe wurde die Säule mit Phosphat-Puffer, pH 7,4, so lange gespült, bis die UV-Absorption des Säuleneffluents den Basislinienwert erreicht hat. Gebundener rvWF wurde durch langsame (5 cm/h) Elution mit 100 mM Glycin, pH 7,4, von der Säule eluiert.

In Tabelle 3 sind die Analysendaten der entsprechenden Fraktionen dargestellt.

**Tabelle 3**

| Probe | vWf Ag (ELISA) | | Ristocetinaktivität | | Protein | | spez. Aktivität | |
|---|---|---|---|---|---|---|---|---|
| | µg/ml | % | U/ml | % | µg/ml | % | mg Ag | U RCoA |
| | | | | | | | mg Prot | mg Prot |
| **Ausgang** | **120** | **100** | **1,5** | **100** | **1400** | **100** | **0,08** | **1,07** |
| **Absorbat** | **10** | **9** | **<0,02** | **<7** | **1220** | **96** | **0,008** | **<0,02** |
| **Eluat** | **370** | **74** | **5,0** | **80** | **350** | **6** | **1,06** | **14,2** |

Aus diesen Ergebnissen ist ersichtlich, daß unter den angegebenen Bedingungen rekombinanter vWF in hoher Reinheit mit einer spezifischen Antigen-Aktivität von mindestens 100 U/mg Protein und mit mindestens 74% Zurückgewinnung in guten Ausbeuten aus Fermentationsüberständen erhalten werden kann.

### Beispiel 4:

### Reinigung von plasmatischem FVIII/vWF-Komplex

50 g Kryopräzipitat wurden in 450 ml Heparinpuffer gelöst. Die Faktoren des Prothrombin-Komplexes wurden durch Zusatz von 0,1% Alhydrogel entfernt. Die geklärte Proteinlösung wird wie in Beispiel 3 beschrieben mittels Immunaffinität gereinigt. In Tabelle 4 sind die Analysendaten vor und nach Reinigung zusammengefaßt.

**Tabelle 4**

| Probe | vWf Ag (ELISA) | | FVIII-Aktivität | | Protein | | spez. Aktivität | |
|---|---|---|---|---|---|---|---|---|
| | µg/ml | % | U/ml | % | mg/ml | % | mg vWF | FVIII:C |
| | | | | | | | mg Prot | mg Prot |
| **Ausgang** | **230** | **100** | **16** | **100** | **21** | **100** | **0,01** | **0,76** |
| **Absorbat** | **10,3** | **6** | **1,1** | **9** | **15,2** | **98** | **0,00** | **0,07** |
| **Eluat** | **320** | **68** | **25,5** | **78** | **0,27** | **0,9** | **1,18** | **94** |

Aus Tabelle 4 ist ersichtlich, daß bei der Verwendung von Kryopräzipitat als Ausgangsmaterial ein FVIII/vWF-Komplex mit hoher Reinheit, einer spezifischen Aktivität von vWF von mindestens 118 Antigen-U/mg Protein und von Faktor VIII von mindestens 94 U/mg gewonnen werden kann. Durch das erfindungsgemäße Verfahren wird freier Faktor VIII entfernt und im wesentlichen reiner Faktor VIII/vWF-Komplex in einem Verhältnis von etwa 1:1 zurückgewonnen Die Ausbeute an reinem Faktor VIII/vWF-Komplex liegt etwa zwischen 70% bis 80% und damit über den bisher beschriebenen Werten für die Proteingewinnung mittels Immunaffinitätschromatographie.

### Beispiel 5:

### Verwendung eines Antikörperfragments für die Immunaffinitätsreinigung von vWF

In gleicher Weise wie der intakte monoklonale Antikörper kann auch ein F'ab- oder F'ab2-Bruchstück des monoklonalen Antikörpers verwendet werden. Diese Vorgangsweise hat den Vorteil, daß eventuell im Endprodukt vorhandene Spuren des Antikörpers keinen immunologisch wirksamen Proteinanteil (Fc-Anteil) mehr enthalten.

Die Fragmentierung des Antikörpers kann durch selektive Proteasen wie Papain oder Pepsin (in freier Form oder an eine unlösliche Trägersubstanz gebunden) erfolgen. Im Folgenden ist die Herstellung, Reinigung und Verwendung eines F'ab-Fragments aus dem monoklonalen Antikörper AvW8-2 beschrieben:

100 mg des monoklonalen Antikörpers AvW8-2 wurden mit 1 mM Cystein versetzt. Zu dieser Lösung wurden 0,5 mg immobilisiertes Papain (Fa. Pierce) zugegeben und die Suspension für 5 Stunden bei 37°C inkubiert. Der Proteaseverdau wurde durch Abfiltrieren des immobilisierten Papains gestoppt, eventuelle Restaktivität wurde durch Zugabe von 10 mM Iodacetamid inhibiert. Die Trennung der F'ab-Fragmente von Fc-Fragmenten, kleineren Bruchstücken und intaktem Antikörper erfolgte mittels Anionentauscherchromatographie an Poros Q. Gereinigte F'ab-Fragmente wurden mit einer Belegungsdichte von 1 mg/ml Gel an CNBr-aktivierte Sepharose gekoppelt. 500 ml eines konzentrierten Fermentationsüberstandes enthaltend r-vWF wurden wie unter Beispiel 3 beschrieben mit 50 ml des so hergestellten Affinitätsharzes gereinigt.

Tabelle 5 zeigt die Ergebnisse der Immunaffinitätschromatographie mittels Elution mit einem Puffer enthaltend 100 mM Glycin, pH 7,4.

**Tabelle 5**

| Probe | vWf Ag (ELISA) | | Ristocetinaktivität | | Protein | | spez. Aktivität | |
|---|---|---|---|---|---|---|---|---|
| | µg/ml | % | U/ml | % | µg/ml | % | mg Ag | U RCoA |
| | | | | | | | mg Prot | mg Prot |
| **Ausgang** | **105** | **100** | **1,8** | **100** | **1250** | **100** | **0,08** | **1,44** |
| **Absorbat** | **9** | **9** | **<0,02** | **<8** | **1200** | **105** | **0,00** | **<0,02** |
| **Eluat** | **248** | **85** | **3,8** | **76** | **265** | **4** | **0,93** | **14.3** |

Aus den Ergebnissen in Tabelle 5 geht hervor, daß sich ein F'ab-Fragment des monoklonalen Antikörpers mit gleicher Effizienz wie der intakte Antikörper für die immunaffinitäts-chromatographische Reinigung von vWF verwenden läßt. Dabei konnte vWF jedoch mit einer verbesserten Ausbeute von 85% des Ausgangsmaterials und einer spezifischen Aktivität von mindestens 93 Antigen-U/mg Protein zurückgewonnen werden.

### Beispiel 6:

### Nachreinigung mittels Heparin-Affinitätschromatographie

Es ist möglich, die Qualität des so gereinigten vWF, sei es durch Anreicherung von vWF-Multimeren bestehend aus hochmolekularen vWF-Multimeren, oder durch Beseitigung von Restkontaminationen, in einer weiteren Reinigungsstufe zu erhöhen. Als Methoden dafür kommen die bekannten Verfahren wie Ionentauscherchromatographie, Heparin-Affinitätschromatographie oder Gelfiltration in Frage.

Im Folgenden ist die Kombination der Immunaffinitätschromatographie mit einer nachfolgenden Heparinchromatographie beschrieben.

Das Eluat der Immunaffinitätschromatographie (Beispiel 5) wird ohne weitere Behandlung auf eine 50 ml-Säule, gefüllt mit Fractogel^{®}-Heparin beladen. Die Elution des gebundenen rvWF erfolgt durch Anlegen eines stufenförmigen Salzgradienten, wobei rvWF vorwiegend in einem Bereich zwischen 0,25 M und 0,3 M NaCl eluiert wird.

Alle Chromatographiepuffer enthalten 100 mM Glycin, um während der Heparinchromatographie die Dissoziation von eventuell ausgebluteten F'ab-Fragmenten von vWF zu gewährleisten. Die Ergebnisse der Heparin-Affinitätschromatographie sind in Tabelle 6 zusammengenfaßt.

**Tabelle 6**

| Probe | vWf Ag (ELISA) | | Ristocetinaktivität | | Protein | | spez. Aktivität | |
|---|---|---|---|---|---|---|---|---|
| | µg/ml | % | U/ml | % | µg/ml | % | mg Ag | U RCoA |
| | | | | | | | mg Prot | mg Prot |
| **Ausgang** | **250** | **100** | **3,8** | **100** | **265** | **100** | **0,94** | **14,3** |
| **Absorbat** | **95** | **46** | **<0,02** | **<4** | **105** | **48** | **0,9** | **<0,19** |
| **Stufe 1** | **85** | **15** | **0,8** | **9** | **89** | **14** | **0,96** | **9,0** |
| **Stufe 2** | **115** | **19** | **7,8** | **85** | **125** | **20** | **0,92** | **62,4** |

Wie aus Tabelle 6 zu ersehen ist, kann durch nachfolgende Reinigungsschritte die Ristocetin-Aktivität des vWF noch weiter verbessert werden. Insbesondere wurde gefunden, daß eine spezifische Plättchenagglutinationsaktivität von über 60 U/mg Protein erhalten wird. Zudem kann über 85% des eingesetzten Materials als gereinigtes Protein mit hoher spezifischer Aktivität wiedergewonnen werden.

Das Endprodukt der Reinigung kann nach der Sterilfiltration und der pharmazeutischen Formulierung direkt in entsprechende Endbehälter verfüllt und lyophilisiert werden. In diesem Falle fungiert das im Puffer enthaltene Glycin als Stabilisierungsmittel während des Lyophilisations- und Lösungsvorganges.

### Beispiel 7:

### Einfluß des Elutionsmittels auf FVIII:C - Aktivität

Gelöstes Kryobulin wurde, wie in Beispiel 4 beschrieben, an einer mit AvW8-2-belegten Säule adsorbiert. Die Kopplung des Antikörpers erfolgte in diesem Fall über Benzochinon, um die pH-Stabilität zu verbessern. Die Elution des gebundenen FVIII/vWF-Komplexes erfolgte einerseits durch pH-Erhöhung wie bei Mejan et al. (1988, Thromb. Haemost. 59: 364-371) beschrieben, andererseits durch Glycin-Elution gemäß dem erfindungsgemäßen Verfahren.

In Tabelle 7 sind die Ergebnisse der Bestimmung der Ausbeute und spezifischen Faktor VIII-Aktivität mittels Immunaffinitätschromatographie mit verschiedenen Elutionsmethoden zusammengestellt.

**Tabelle 7**

| Elutionsmethode | spez. Aktivität U FVIII:C mg Protein | Ausbeute FVIII:C % |
|---|---|---|
| pH - Shift | 37 | 21 |
| Glycin | 67 | 82 |

Aus Tabelle 7 ist ersichtlich, daß bei Verwendung von Glycin-Puffer als Elutionsmittel die Ausbeute bis auf das 4fache gegenüber dem pH-shift erhöht wird und die spezifische Aktivität des gewonnenen Faktor VIII um das 2fache verbessert ist. Der Grund dafür dürfte die Exposition bzw. Aktivierung des FVIII bei einem pH-shift sein, während bei milden Elutionsbedingungen bei neutralem pH in Anwesenheit einer Aminosäure bzw. des Zwitterions die Aktivität von Faktor VIII weniger beeinträchtigt wird.

### Beispiel 8:

### Reinigung eines Komplexes bestehend aus rekombinantem FVIII und rekombinantem vWF

Zellkulturüberstände aus rekombinanten Zellen transfiziert mit einem Vektor enthaltend cDNA kodierend für Faktor VIII bzw. vWF wurden in einem Verhältnis 3 Faktor VIII: 1 vWF gemischt. Die Mischung enthaltend rFVIII/rvWF-Komplex wurde unter Bedingungen wie in Beispiel 3 angegeben gereinigt.

In Tabelle 8 sind die Ergebnisse dieser Reinigung von rFaktor VIII/rvWF dargestellt.

**Tabelle 8**

| Probe | U FVIII:C/ml | Ausbeute FVIII:C | µg VWF/ml | Ausbeute vWF | U FVIII:C U vWF |
|---|---|---|---|---|---|
| Auftrag | 2,1 | 100 | 6,1 | 100 | 3,4 |
| Durchfluß | 0,4 | 19 | neg. | 0 | n.d. |
| Glycin-Eluat | 8,3 | 73 | 25,7 | 67 | 3,2 |

Tabelle 8 zeigt, daß sich ein Komplex bestehend aus rFVIII und rvWF unter den angegebenen Bedingungen gleich wie ein plasmatischer Komplex verhält. Ebenso wurde abhängig vom Mischungsverhältnis des Ausgangsmaterials rFVIII/rvWF-Komplex in einem bestimmten Verhältnis zueinander wiedergewonnen. Durch Variation des FVIII/vWF-Verhältnisses im Ausgang oder durch Anschließen eines weiteren chromatographischen Schrittes kann das Verhältnis von FVIII:C zu vWF noch zugunsten des relativen FVIII-Gehalts variiert werden. Dadurch ist es möglich, gezielt einen Faktor VIII/vWF-Komplex mit einem bestimmten gewünschten Verhältnis zu erhalten.

### Beispiel 9:

### Test auf Maus-IgG (F'ab-Fragmente)

Kontaminierende F'ab-Fragmente im Eluat der Immunaffinitätssäule werden mit Hilfe eines immunologischen Verfahrens nachgewiesen (Immuno Ligand Assay, Molecular Devices).

Kaninchen Anti-Maus IgG (F'ab-spezifisch) wird mit Biotin oder Fluorescein markiert. 100 µl des Säuleneluates werden mit jeweils 2 µg des biotin- und fluoresceinmarkierten Antikörpers gemischt und inkubiert. Nach einer weiteren Inkubation mit 2 µg Streptavidin und ureasemarkierten anti-Fluorescein-Antikörpern wird das Gemisch durch eine biotinylierte Membran filtriert. Nach dem Waschen der Membran kann gebundenes F'ab-Fragment mit hoher Empfindlichkeit (ca. 100 pg/ml) nachgewiesen werden.

In Tabelle 9 sind die Ergebnisse unterschiedlicher Chromatographieläufe zusammengefaßt.

**Tabelle 9**

| Probe | Elutionsmethode | vWF-Antigen | Maus IgG | ng Maus IgG |
|---|---|---|---|---|
| | | µg/ml | ng/ml | mg vWF Antigen |
| Kryopräzipitat | pH-Shift * | 280 | 34,5 | 123,2 |
| rekomb. vWF | pH-Shift * | 190 | 28,6 | 150,5 |
| Kryopräzipitat | Glycin | 320 | 4,2 | 13,1 |
| rekomb. vWF | Glycin | 248 | 1,9 | 7,6 |

| | | | | |
|---|---|---|---|---|
| * Die Kopplung des Antikörpers erfolgte in diesem Fall über Benzochinon, um eine bessere pH-Stabilität zu erhalten | | | | |

Aus Tabelle 9 ist zu erkennen, daß es mit dem erfindungsgemäßen Verfahren möglich ist, vWF bzw. Faktor VIII/vWF-Komplex mit sehr geringen Mengen an kontaminierendem Maus-IgG (F'ab-Fragmenten) zu gewinnen.

Durch Anfügen eines weiteren chromatographischen Reinigungsschrittes (Heparinchromatographie oder Ionentauscherchromatographie) ist es möglich, diese Restkontamination unter die Nachweisgrenze zu reduzieren.

### Beispiel 10:

### Integrität des gereinigten FVIII/vWF-Komplexes

Um die Integrität des gereinigten Komplexes, bestehend aus FVIII und vWF zu demonstrieren, wurden 5 ml des Eluats der Immunaffinitätssäule erhalten nach Beispiel 4 einer Gelfiltrationssäule, gefüllt mit 120 ml Sepharose CL6B aufgetragen. Durch Elution mit Glycinpuffer als Laufmittel erfolgt eine Trennung nach der Molekülgröße während des Chromatographielaufes.

Aufgund der Trenncharakteristik des Säulenmaterials würde es bei Vorliegen zweier unterschiedlicher Molekülspezies (FVIII und vWF) zu einer Separation der beiden Proteine kommen. In Fig. 1 sind die Ergebnisse dieses Chromatographielaufes dargestellt.

Man erkennt deutlich, daß der nach Beispiel 4 erhaltene Komplex aus FVIII und vWF in einem Peak erscheint ( 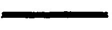 ). Wären die beiden Proteine nicht assoziiert, würden sie deutlich voneinander getrennt eluieren (siehe Markierung).

FVIII/vWF-Komplex nach dem erfindungsgemäßen Verfahren gereinigt liegt also als Proteinkomplex vor, in welchem der vWF die Aufgabe der Stabilisierung des FVIII übernimmt.

## Patentansprüche

1. Verfahren zur Gewinnung von hochreinem vWF bzw. Faktor VIII/vWF-Komplex mittels Immunaffinitätschromatographie, wobei ein an ein Immunadsorbens gebundener vWF bzw. Faktor VIII/vWF-Komplex mit einem Elutionsmittel enhaltend als wesentlichen aktiven Bestandteil ein Zwitterion unter Erhaltung der molekularen Integrität des vWF bzw. des Faktor VIII/vWF-Komplexes eluiert wird, **dadurch gekennzeichnet, dass** das Zwitterion eine Aminosäure oder ein Aminosäureanalog mit einem isoelektrischen Punkt zwischen 3,2 und 9,6 ist und dass der an Immunadsorbens gebundene vWF bzw. Faktor VIII/vWF-Komplex ohne die Verwendung von chaotropen Reagentien dissoziiert wird.

2. Verfahren nach Anspruch 1, **dadurch gekennzeichnet, dass** das Zwitterion ausgewählt ist aus der Gruppe von neutralen Aminosäuren, insbesondere Glycin, Alanin, β-Alanin, Phenylalanin und Histidin, oder Betainen.

3. Verfahren nach einem der Ansprüche 1 oder 2, **dadurch gekennzeichnet, dass** das Zwitterion in der Lösung in einer Konzentration zwischen 0,01 und 0,5 M, vorzugsweise zwischen 0,08 und 0,2 M, besonders bevorzugt zwischen 0,1 und 0,15 M vorliegt.

4. Verfahren nach einem der Ansprüche 1 bis 3, **dadurch gekennzeichnet, dass** das Elutionsmittel einen physiologischen pH Wert aufweist, vorzugsweise zwischen pH 7,0 und pH 8,0, besonders bevorzugt zwischen pH 7,3 und pH 7,8, insbesondere bevorzugt von pH 7,4.

5. Verfahren nach einem der Ansprüche 1 bis 4, **dadurch gekennzeichnet, dass** das Immunadsorbens ein anti-vWF-Antikörper, vorzugsweise ein monoklonaler Antikörper ist.

6. Verfahren nach einem der Ansprüche 1 bis 4, **dadurch gekennzeichnet, dass** das Immunadsorbens ein Fragment eines anti-vWF-Antikörpers ist.

7. Verfahren nach einem der Ansprüche 1 bis 6, **dadurch gekennzeichnet, dass** gereinigter vWF oder Faktor VIII/vWF-Komplex in einer wässrigen Lösung enthaltend ein Zwitterion in einer Konzentration zwischen 0,08 M und 0,2 M und einem pH zwischen 7,3 und 7,8 gewonnen wird, wobei das Zwitterion ausgewählt ist aus der Gruppe der Aminosäuren Glycin, Alanin, β-Alanin, Phenylalanin und Histidin, oder der Betaine.

8. Verfahren nach einem der Ansprüche 1 bis 7, **dadurch gekennzeichnet, dass** gereinigter vWF oder Faktor VIII/vWF-Komplex einem weiteren chromatographischen Schritt, vorzugsweise eine Heparinaffinitätschromatographie unterzogen wird.

9. Verfahren nach einem der Ansprüche 7 oder 8, **dadurch gekennzeichnet, dass** gereinigter vWF oder Faktor VIII/vWF-Komplex ohne Zusatz von hochmolekularen Stabilisatoren lyophilisiert wird.

10. Verfahren nach einem der Ansprüche 1 bis 8, **dadurch gekennzeichnet, dass** gereinigter vWF oder Faktor VIII/vWF-Komplex einem Virusinaktivierungs- und/oder Virusabreicherungsschritt unterzogen wird.

## Claims

1. A method of recovering highly purified vWF or factor VIII/vWF-complex, respectively, by means of immunoaffinity chromatography, wherein vWF, or a factor VIII/vWF-complex, respectively, bound to an immunoadsorbent is eluted by means of an eluting agent containing, as an essential active component, a zwitterion, whereby the molecular integrity of the vWF, or of the factor VIII/vWF-complex, respectively, is retained, **characterised in that** the zwitter ion is an amino acid or an amino acid analogue having an isoelectric point of between 3.2 and 9.6 and **in that** the vWF, or the factor VIII/vWF-complex, respectively, bound to the immunoadsorbent is dissociated without the use of chaotropic reagents.

2. A method according to claim 1, **characterised in that** the zwitterion is selected from the group of neutral amino acids, in particular glycine, alanine, β-alanine, phenylalanine and histidine, or betaines.

3. A method according to any one of claims 1 or 2, **characterised in that** the zwitterion in the solution is present at a concentration of between 0.01 and 0.5 M, preferably between 0.08 and 0.2 M, particularly preferred between 0.1 and 0.15 M.

4. A method according to any one of claims 1 to 3, **characterised in that** the eluting agent has a physiological pH, preferably of between pH 7.0 and pH 8.0, particularly preferred between pH 7.3 and pH 7.8, most particularly preferred of pH 7.4.

5. A method according to any one of claims 1 to 4, **characterised in that** the immunoadsorbent is an anti-vWF-antibody, preferably a monoclonal antibody.

6. A method according to any one of claims 1 to 4, **characterised in that** the immunoadsorbent is a fragment of an anti-vWF-antibody.

7. A method according to any one of claims 1 to 6, **characterised in that** purified vWF or factor VIII/vWF-complex is recovered in an aqueous solution containing a zwitterion at a concentration of between 0.08 M and 0.2 M and having a pH of between 7.3 and 7.8, the zwitterion being selected from the group of the amino acids glycine, alanine, β-alanine, phenlyalanine and histidine, or of the betaines.

8. A method according to any one of claims 1 to 7, **characterised in that** purified vWF or factor VIII/vWF-complex is subjected to a further chromatographic step, preferably a heparin affinity chromatography.

9. A method according to any one of claims 7 or 8, **characterised in that** purified vWF or factor VIII/vWF-complex is lyophilized without the addition of high-molecular stabilizers.

10. A method according to any one of claims 1 to 8, **characterised in that** the purified vWF or factor VIII/vWF-complex is subjected to a virus inactivation- and/or virus depletion step.

## Revendications

1. Procédé permettant d'obtenir un facteur de von Willebrand (vWF) ou un complexe facteur VIII / vWF hautement purifié par chromatographie d'immunoaffinité, un vWF ou un complexe facteur VIII / vWF lié à un immunoadsorbant étant élué avec un agent d'élution comprenant comme composant actif principal un ion amphotère, en conservant l'intégrité moléculaire du vWF ou du complexe de facteur VIII / vWF, **caractérisé en ce que** l'ion amphotère est un acide aminé ou un analogue d'acide aminé présentant un point isoélectrique compris entre 3,2 et 9,6, et **en ce que** le vWF ou le complexe facteur VIII / vWF lié à un immunoadsorbant est dissocié sans l'utilisation de réactif chaotrope.

2. Procédé selon la revendication 1, **caractérisé en ce que** l'ion amphotère est choisi parmi le groupe composé d'acides aminés neutres, en particulier la glycine, l'alanine, la β-alanine, la phénylalanine et l'histidine, ou de bétaïnes.

3. Procédé selon l'une quelconque des revendications 1 ou 2, **caractérisé en ce que** l'ion amphotère est présent dans la solution à une concentration comprise entre 0,01 et 0,5 M, de préférence entre 0,08 et 0,2 M, de manière particulièrement préférée entre 0,1 et 0,15 M.

4. Procédé selon l'une quelconque des revendications 1 à 3, **caractérisé en ce que** l'agent d'élution présente une valeur de pH physiologique, comprise de préférence entre pH 7,0 et pH 8,0, de manière particulièrement préférée entre pH 7,3 et pH 7,8, et en particulier de manière préférée entre toutes de pH 7,4.

5. Procédé selon l'une quelconque des revendications 1 à 4, **caractérisé en ce que** l'immunoadsorbant est un anticorps anti-vWF, de préférence un anticorps monoclonal.

6. Procédé selon l'une quelconque des revendications 1 à 4, **caractérisé en ce que** l'immunoadsorbant est un fragment d'un anticorps anti-vWF.

7. Procédé selon l'une quelconque des revendications 1 à 6, **caractérisé en ce que** le vWF ou le complexe facteur VIII / vWF est obtenu dans une solution aqueuse comprenant un ion amphotère à une concentration comprise entre 0,08 M et 0,2 M et à un pH compris entre 7,3 et 7,8, l'ion amphotère étant choisi parmi le groupe composé des acides aminés glycine, alanine, β-alanine, phénylalanine et histidine, ou de bétaïnes.

8. Procédé selon l'une quelconque des revendications 1 à 7, **caractérisé en ce que** le vWF ou le complexe facteur VIII / vWF purifié est soumis à une étape chromatographique supplémentaire, de préférence par chromatographie d'affinité sur héparine.

9. Procédé selon l'une quelconque des revendications 7 ou 8, **caractérisé en ce que** le vWF ou le complexe facteur VIII / vWF purifié est lyophilisé sans l'ajout de stabilisateurs à poids moléculaire élevé.

10. Procédé selon l'une quelconque des revendications 1 à 8, **caractérisé en ce que** le vWF ou le complexe facteur VIII / vWF purifié est soumis à une étape d'inactivation virale et/ou d'appauvrissement viral.
